# EUROPÄISCHE PATENTSCHRIFT

(11) **EP 2 242 872 B1**
(45) Veröffentlichungstag und Bekanntmachung des Hinweises auf die Patenterteilung: **07.12.2011**
(21) Anmeldenummer: 08869797.4
(22) Anmeldetag: 10.12.2008
(51) Int. Cl.: C23F 11/10, C23F 11/14, C09K 8/528, C09K 8/54, C10M 133/44, C07D 207/277

(54) **KORROSIONSINHIBITOREN MIT ERHÖHTER BIOLOGISCHER ABBAUBARKEIT UND VERMINDERTER TOXIZITÄT**
CORROSION INHIBITORS HAVING INCREASED BIOLOGICAL DEGRADABILITY AND MINIMIZED TOXICITY
INHIBITEURS DE CORROSION PRÉSENTANT UNE MEILLEURE BIODÉGRADABILITÉ ET UNE TOXICITÉ RÉDUITE

(30) Priorität: 10.01.2008 DE 102008003826
(43) Veröffentlichungstag der Anmeldung: 27.10.2010
(73) Patentinhaber: Clariant Finance (BVI) Limited, Road Town, Tortola (VG)
(72) Erfinder: LEINWEBER, Dirk, 65779 Kelkheim (DE); RÖSCH, Alexander, 67578 Gimbsheim (DE); FEUSTEL, Michael, 55278 Köngernheim (DE)
(74) Vertreter: Mikulecky, Klaus
(86) Internationale Anmeldenummer: PCT/EP2008/010458
(87) Internationale Veröffentlichungsnummer: WO 2009/086873

(56) Entgegenhaltungen:
- DE-A1- 3 701 494
- GB-A- 1 323 061
- US-A- 3 051 722
- US-A- 3 224 968

## Beschreibung

Die vorliegende Erfindung betrifft ein Verfahren zur Korrosionsinhibierung an und in Einrichtungen zur Förderung und Transport von Kohlenwasserstoffen in der Erdölförderung und -verarbeitung, indem man dem korrosiven System ein Salz aus einer Stickstoffbase und einer N-substituierten 5-Oxo-pyrrolidin-3-carbonsäure zusetzt.

In technischen Prozessen, bei denen Metalle mit Wasser oder auch mit ÖI-Wasser-Zweiphasensystemen in Kontakt kommen, besteht die Gefahr der Korrosion. Diese ist besonders ausgeprägt, wenn die wässrige Phase wie bei Erdölgewinnungs- und Verarbeitungsprozessen stark salzhaltig oder durch gelöste (Sauer)-Gase, wie Kohlendioxid bzw. Schwefelwasserstoff, azide ist. Daher sind die Ausbeutung einer Lagerstätte und die Verarbeitung von Erdöl ohne spezielle Additive zum Schutz der eingesetzten Ausrüstungen nicht möglich.

Geeignete Korrosionsschutzmittel für die Erdölförderung und -verarbeitung sind zwar schon seit langem bekannt, jedoch aus Gründen des Umweltschutzes für Offshore-Anwendungen zukünftig inakzeptabel.

Als typische Korrosionsinhibitoren des Standes der Technik besitzen Amide, Amidoamine bzw. Imidazoline von Fettsäuren und Polyaminen eine äußerst gute Öllöslichkeit und sind somit in der korrosiven Wasserphase aufgrund schlechter Verteilungsgleichgewichte (Partitioning) nur in geringer Konzentration vorhanden. Demgemäß müssen diese Produkte trotz ihrer schlechten biologischen Abbaubarkeit in hoher Dosierung eingesetzt werden.

Quartäre Alkylammoniumverbindungen (Quats) stellen alternative Korrosionsschutzmittel des Standes der Technik dar, die neben den korrosionsinhibierenden auch biostatische Eigenschaften besitzen können. Trotz einer verbesserten Wasserlöslichkeit zeigen die Quats, zum Beispiel im Vergleich zu den Imidazolinen, eine deutlich reduzierte Filmpersistenz und führen daher ebenfalls nur in höherer Dosierung zu einem effektiven Korrosionsschutz. Die starke Algentoxizität und die mäßige biologische Abbaubarkeit beschränken den Einsatz von Quats immer mehr auf ökologisch unsensible Anwendungsgebiete.

US-2 757 125 beschreibt Salze von N-alkyl-4-carboxy-2-pyrrolidonen, die als antibakterielle Komponenten in kosmetischen Formulierungen oder Reinigungsmitteln eingesetzt werden. Die Verwendung dieser Verbindungen als Korrosionsinhibitoren im Bereich Ölfeldchemikalien wird nicht beschreiben.

In US-2 908 711 und US-3 035 907 werden öllösliche Umsetzungsprodukte aus Aminen bzw. Diaminen und Itaconsäure beschrieben, die als Anti-Rost Additive in Treibstoffen oder Mineralölen eingesetzt werden können.

In US-3 218 264 werden öllösliche Pyrrolidoncarbonsäure-Aminsalze und deren Verwendungen als Korrosionsinhibitoren in Schmierölen und- fetten offenbart. Die zur Salzbildung verwendeten Amine sind erfindungsgemäß öllöslich.

In US-3 224 968 werden ebenfalls öllösliche Aminsalze von Pyrrolidoncarbonsäuren beschrieben, die Verwendung als Anti-Rost Additive in Schmierölen Verwendung finden. Wiederum werden zur Aminsalz-Bildung öllösliche Amine (bevorzugt C₁₂-C₂₀-Alkyl substituiert) eingesetzt. US-3 224 975 beschreibt die freien Pyrrolidoncarbonsäuren für die gleiche Verwendung.

GB-A-1 323 061 offenbart Pyrrolidonderivate und deren Verwendung in Funktionsflüssigkeiten wie beispielsweise Hydraulikflüssigkeiten. Die verwendeten Verbindungen weisen C₁-C₅-Alkylsubstituenten bzw. C₆-C₁₀-Arylsubstituenten am Pyrrolidon-Stickstoff auf. In Hydraulikflüssigkeiten zeigen die Verbindungen antikorrosive Eigenschaften, auch in Kombination mit aliphatischen Aminen.

In EP-A-0 069 512 werden wasserlösliche Salze von N-substituierten-2-pyrrolidon-4-carbonsäuren als Befeuchtungsmittel ("Humectants") beschrieben.

Aufgabe der vorliegenden Erfindung war es, neue Korrosionsinhibitoren zu finden, die bei konstant gutem oder verbessertem Korrosionsschutz neben einer guten Wasserlöslichkeit, auch eine verbesserte biologische Abbaubarkeit und niedrigere Toxizität im Vergleich zu den Korrosionsinhibitoren des Standes der Technik bieten.

Es wurde nun überraschenderweise gefunden, dass wasserlösliche bzw. wasserdispergierbare Salze bestimmter cyclischer Amine und einer N-substituierten 5-Oxo-pyrrolidin-3-carbonsäure eine ausgezeichnete Wirkung als Korrosionsinhibitoren, sowie eine gute biologische Abbaubarkeit und verminderte Toxizität zeigen.

Gegenstand der Erfindung ist somit die Verwendung von Salzen aus Verbindungen der Formel (1) 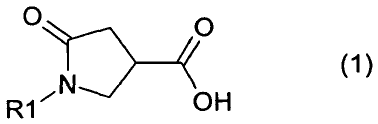 mit Aminen der Formel (2) 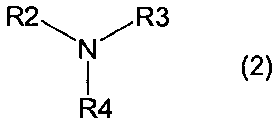 worin
- R1: C₈- bis C₃₀-Alkyl oder C₈- bis C₃₀-Alkenyl
- R2: Wasserstoff, C₁- bis C₆-Alkyl oder einen Heteroatome enthaltenden organischen Rest mit 1 bis 8 Kohlenstoffatomen, und,
- R3, R4: unter Einschluss des Stickstoffatoms einen Cyclus mit 5 bis 7 Ringatomen bilden, wobei dieser Cyclus neben Kohlenstoffatomen gegebenenfalls ein Sauerstoffatom oder ein Stickstoffatom oder beides enthalten kann,
als wasserlösliche bzw. wasserdispergierbare Korrosionsinhibitoren.

Ein weiterer Gegenstand der Erfindung sind Salze aus Verbindungen der Formel (1) und Aminen der Formel (2).

Ein weiterer Gegenstand der Erfindung ist ein Verfahren zur Inhibierung von Korrosion an Metalloberflächen, insbesondere von eisenhaltigen Metallen, indem einem korrosiven System, welches mit den Metalloberflächen in Kontakt steht, mindestens ein Salz Verbindungen der Formel (1) und Aminen der Formel (2) zugesetzt wird.

Ein weiterer Gegenstand der Erfindung ist die Verwendung der Verbindungen der Formeln (1) und (2) als Metallbearbeitungsmittel. Hierbei bieten die erfindungsgemäßen Verbindungen auch bei starker mechanischer Belastung, wie beim Schleifen, Schneiden und Bohren von Metallwerkstücken einen sehr guten Korrosionsschutz.

Korrosive Systeme im Sinne dieser Erfindung sind bevorzugt flüssig/flüssig- bzw. flüssig/gasförmig-Mehrphasensysteme, bestehend aus Wasser und Kohlenwasserstoffen, die in freier und/oder gelöster Form korrosive Bestandteile, wie Salze und Säuren, enthalten. Die korrosiven Bestandteile können auch gasförmig sein, wie etwa Schwefelwasserstoff und Kohlendioxid.

Kohlenwasserstoffe im Sinne dieser Erfindung sind organische Verbindungen, die Bestandteile des Erdöls/Erdgases sind, und deren Folgeprodukte. Kohlenwasserstoffe im Sinne dieser Erfindung sind auch leichtflüchtige Kohlenwasserstoffe, wie beispielsweise Methan, Ethan, Propan, Butan. Für die Zwecke dieser Erfindung zählen dazu auch die weiteren gasförmigen Bestandteile des Erdöls/Erdgases, wie etwa Schwefelwasserstoff und Kohlendioxid.

Bei den Aminen der Formel 2 handelt es sich vorzugsweise um Destillationsrückstände der Morpholin-Herstellung (Morpholin-Prozessrückstände), welche Stoffgemische der Nummer CAS 68909-77-3 sind.

Bei Destillationsrückständen der Morpholin-Herstellung (CAS 68909-77-3) handelt es sich um einen Rückstand aus der Reaktion von Diethylenglykol und Ammoniak. Er besteht vorherrschend aus Derivaten auf Morpholinbasis wie [(Aminoethoxy)ethyl]-morpholin, [(Hydroxyethoxy)ethyl]morpholin, 3-Morpholinon und 4,4'-(Oxydi-2,1-ethandiyl)bis[morpholin], also Verbindungen der Formeln 3-6:

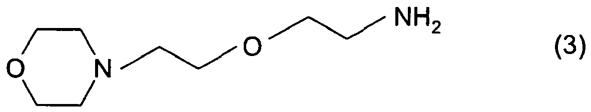

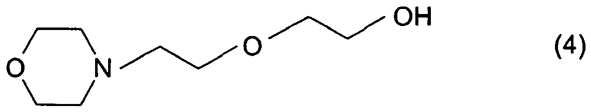

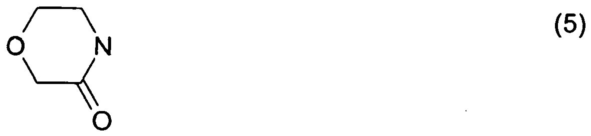

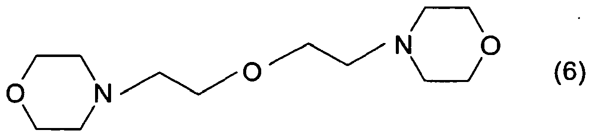

Vorzugsweise ist die Verbindung der Formel 2 aus den Verbindungen der Formeln 3 bis 6 ausgewählt.

In Formel (1) steht R1 vorzugsweise für eine Alkyl- oder Alkenylgruppe mit 8 bis 24 Kohlenstoffatomen, insbesondere für eine Alkyl- oder Alkenylgruppe mit 8 bis 18 Kohlenstoffatomen. Besonders bevorzugt steht R1 für einen Octyl-, Kokoyloder Oleylrest.

R2 steht für vorzugsweise für Wasserstoff oder einen organischen Rest mit 1 bis 4 C-Atomen und gegebenenfalls Sauerstoff- oder Stickstoffatome enthalten kann. In einer besonders bevorzugten Ausführungsform steht R2 für -CH₂-CH₂-OH.

R3 und R4 enthalten neben dem in Formel 2 abgebildeten Stickstoffatom Kohlenstoffatome, und gegebenenfalls ein Sauerstoffatom oder ein weiteres Stickstoffatom oder beides. Die Gesamtzahl der Ringatome ist vorzugsweise gleich sechs.

In einer weiteren bevorzugten Ausführungsform der Erfindung ist die Verbindung der Formel (2) ein cyclisches Amin der Formel (7) 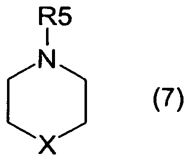 worin
- R5: Wasserstoff oder einen gegebenenfalls Heteroatome enthaltenden C₁-C₆-Alkylrest, und
- X: C, O oder N bedeuten.

Die erfindungsgemäßen Verbindungen können alleine oder in Kombination mit anderen bekannten Korrosionsinhibitoren eingesetzt werden. Im Allgemeinen wird man so viel des erfindungsgemäßen Korrosionsinhibitors einsetzen, dass man unter den gegebenen Bedingungen einen ausreichenden Korrosionsschutz erhält.

Bevorzugte Einsatzkonzentrationen der Korrosionsinhibitoren bezogen auf die reinen erfindungsgemäßen Salze sind 5 bis 5.000 ppm, bevorzugt 10 bis 1.000, insbesondere 15 bis 150 ppm.

Die Herstellung der N-substituierten 5-Oxo-pyrrolidin-3-carbonsäuren erfolgt wie im Stand der Technik ausführlich beschrieben durch Umsetzung von Itaconsäure mit primären Aminen und kann wie in EP-A-0 069 512, US-3 224 975 und US-4 127 493 beschrieben durchgeführt werden.

Die Reinheit der so erhaltenen N-substituierten 5-Oxo-pyrrolidin-3-carbonsäuren beträgt im Allgemeinen 80-100 %, im Besonderen 88-98 % und speziell 90-95 %.

Die erfindungsgemäßen Salze aus den Formeln (1) und (2) werden durch eine Neutralisationsreaktion entweder in Substanz oder in einem geeigneten Lösungsmittelsystem, bevorzugt Mischungen aus Wasser und einem Alkohol, hergestellt. Dabei wird bevorzugterweise das Amin der Formel (2) im Lösungsmittel gelöst und die N-substituierte 5-Oxo-pyrrolidin-3-carbonsäure unter Rühren hinzugegeben.

### Beispiele:

### Allgemeine Vorschrift für die Herstellung von N-substituierten 5-Oxo-pyrrolidin-3-carbonsäure Ammoniumsalzen

In einer Standard-Rührapparatur werden 1 mol Amin vorgelegt und unter Rühren auf 50 °C erwärmt. Nun werden portionsweise 1 mol Itaconsäure zugegeben und die Reaktionsmischung langsam auf 180 °C erwärmt. Während des Fortschreitens der Reaktion wird 1 mol Reaktionswasser abdestilliert. Anschließend wird die N-substituierte 5-Oxo-pyrrolidin-3-carbonsäure durch Zugabe einer äquimolaren Menge des entsprechenden Amins in das N-substituierte 5-Oxo-pyrrolidin-3-carbonsäure Ammoniumsalz überführt. Das erhaltene Produkt wird mittels Säurezahl (SZ) und Basen-Stickstoff (bas.-N) charakterisiert. Prozentangaben sind Gewichtsprozente bezogen auf das Gewicht des erfindungsgemäßen Salzes.

### Beispiel 1

### N-Kokoyl-5-oxo-pyrrolidin-3-carbonsäure, Ammoniumsalz mit Morpholin Prozessrückständen

Aus 196 g Kokosfettamin, 130 g Itaconsäure und 127 g Morpholin Prozessrückständen wurden 435 g N-Kokoyl-5-oxo-pyrrolidin-3-carbonsäure, Ammoniumsalz mit Morpholin Prozessrückständen mit SZ = 123 mg KOH/g und bas.-N = 3,2 % erhalten.

### Beispiel 2

### N-Oleyl-5-oxo-pyrrolidin-3-carbonsäure, Ammoniumsalz mit Morpholin Prozessrückständen

Aus 265 g Oleylamin, 130 g Itaconsäure und 127 g Morpholin Prozessrückständen wurden 506 g N-Oleyl-5-oxo-pyrrolidin-3-carbonsäure, Ammoniumsalz mit Morpholin Prozessrückständen mit SZ = 110 mg KOH/g und bas.-N = 2,8 % erhalten.

### Beispiel 3

### N-Octyl-5-oxo-pyrrolidin-3-carbonsäure, Ammoniumsalz mit Morpholin Prozessrückständen

Aus 129 g Octylamin, 130 g Itaconsäure und 127 g Morpholin Prozessrückständen wurden 369 g N-Octyl-5-oxo-pyrrolidin-3-carbonsäure, Ammoniumsalz mit Morpholin Prozessrückständen mit SZ = 151 mg KOH/g und bas.-N = 3,8 % erhalten.

Wirksamkeit der erfindungsgemäßen Verbindungen als Korrosionsinhibitoren

Die erfindungsgemäßen Verbindungen wurden als Korrosionsinhibitoren im Shell-Wheel-Test geprüft. Coupons aus C-Stahl (DIN 1.1203 mit 15 cm² Oberfläche) wurden in eine Salzwasser/Petroleum-Mischung (9:1,5 %ige NaCl-Lösung mit Essigsäure auf pH 3,5 gestellt) eingetaucht und bei einer Umlaufgeschwindigkeit von 40 rpm bei 70 °C 24 Stunden diesem Medium ausgesetzt. Die Dosierung des Inhibitors betrug 50 ppm einer 40 % Lösung des Inhibitors. Die Schutzwerte wurden aus der Massenabnahme der Coupons, bezogen auf einen Blindwert, berechnet.

In den folgenden Tabellen bezeichnet "Vergleich 1" ein handelsübliches Rückstandsamin-Quat auf Basis Dikokosalkyldimethylammoniumchlorid, "Vergleich 2" ein Beispiel aus US-3 224 975 (N-Oleyl-5-oxo-pyrrolidin-3-carbonsäure, Korrosionsinhibitor des Standes der Technik), "Vergleich 3" ein Beispiel aus US-3 224 968 (N-Oleyl-5-oxo-pyrrolidin-3-carbonsäure, Salz mit Oleylamin, Korrosionsinhibitor des Standes der Technik) und "Vergleich 4" ein Beispiel aus GB-1 323 061 (N-Butyl-5 oxo-pyrrolidin-3-carbonsäure, Salz mit Dibutylamin, Korrosionsinhibitor des Standes der Technik).

**Tabelle 1: (Shell-Wheel-Test)**

| Beispiel | Korrosionsinhibitor | Ø Schutz % |
|---|---|---|
| Vergleich 1 | Standard-Quat | 36 |
| Vergleich 2 | N-Oleyl-5-oxo-pyrrolidin-3-carbonsäure | 60 |
| Vergleich 3 | N-Oleyl-5-oxo-pyrrolidin-3-carbonsäure, Salz mit Oleylamin | 55 |
| Vergleich 4 | N-Butyl-5-oxo-pyrrolidin-3-carbonsäure, Salz mit Dibutylamin | 21 |
| 2 | Verbindung aus Beispiel 1 | 88 |
| 3 | Verbindung aus Beispiel 2 | 90 |
| 4 | Verbindung aus Beispiel 3 | 76 |

Die Produkte wurden außerdem im LPR-Test (Testbedingungen analog ASTM D 2776) geprüft.

**Tabelle 2: (LPR-Test)**

| Beispiel | Korrosionsinhibitor | Schutz nach [%] | | |
|---|---|---|---|---|
| | | 10 min | 30 min | 60 min |
| Vergleich 1 | Standard-Quat | 54 | 61 | 74 |
| Vergleich 2 | N-Oleyl-5-oxo-pyrrolidin-3-carbonsäure | 2 | 10 | 23 |
| Vergleich 3 | N-Oleyl-5-oxo-pyrrolidin-3-carbonsäure, Salz mit Oleylamin | 1 | 8 | 17 |
| Vergleich 4 | N-Butyl-5-oxo-pyrrolidin-3-carbonsäure, Salz mit Dibutylamin | 4 | 12 | 25 |
| 5 | Verbindung aus Beispiel 1 | 90 | 95 | 99 |
| 6 | Verbindung aus Beispiel 2 | 88 | 94 | 99 |
| 7 | Verbindung aus Beispiel 3 | 75 | 83 | 90 |

Wie aus den obigen Testresultaten zu erkennen ist, weisen die erfindungsgemäßen Produkte sehr gute Korrosionsschutzeigenschaften bei niedriger Dosierung auf und übertreffen die Wirksamkeit der Inhibitoren des Standes der Technik deutlich.

**Tabelle 3:**

| Biologische Abbaubarkeit (OECD 306) und Toxizität (EC₅₀ Skeletonema Costatum) | | | |
|---|---|---|---|
| Beispiel | Korrosionsinhibitor | Biologische Abbaubarkeit [%] | Toxizität EC₅₀ [mg/L] |
| Vergleich 1 | Standard-Quat | 15 | <1 |
| 8 | Verbindung aus Beispiel 1 | 74 | >100 |
| 9 | Verbindung aus Beispiel 2 | 70 | >100 |
| 10 | Verbindung aus Beispiel 3 | 83 | >100 |

Wie aus Tabelle 4 klar ersichtlich ist, zeigen die erfindungsgemäßen Verbindungen eine bessere biologische Abbaubarkeit und niedrigere Toxizität als das Vergleichsbeispiel aus dem Stand der Technik.

## Patentansprüche

1. Verwendung von Salzen aus Verbindungen der Formel (1) 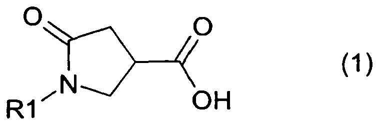 mit Aminen der Formel (2) 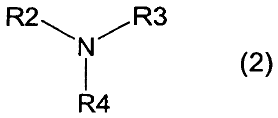 worin
R1 C₈- bis C₃₀-Alkyl oder C₈- bis C₃₀-Alkenyl
R2 Wasserstoff, C₁- bis C₆-Alkyl oder einen Heteroatome enthaltenden organischen Rest mit 1 bis 8 Kohlenstoffatomen, und,
R3, R4 unter Einschluss des Stickstoffatoms einen Cyclus mit 5 bis 7 Ringatomen bilden, wobei dieser Cyclus neben Kohlenstoffatomen gegebenenfalls ein Sauerstoffatom oder ein Stickstoffatom oder beides enthalten kann,
bedeuten, als wasserlösliche bzw. wasserdispergierbare Korrosionsinhibitoren.

2. Verwendung nach Anspruch 1, wobei R1 für eine Alkyl- oder Alkenylgruppe von 8 bis 18 Kohlenstoffatomen steht.

3. Verwendung nach Anspruch 1 und/oder 2, wobei R2 für -CH₂-CH₂-OH steht.

4. Verwendung nach einem oder mehreren der Ansprüche 1 bis 3, worin das Amin der Formel (2) eine Verbindung der Formel (7) ist 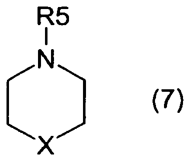 worin
R5 Wasserstoff oder einen gegebenenfalls Heteroatome enthaltenden C₁ bis C₆ Alkylrest, und
X C, O oder N bedeuten.

5. Verwendung nach einem oder mehreren der Ansprüche 1 bis 3, worin das Amin der Formel 2 ein Destillationsrückstand der Morpholin-Herstellung ist.

6. Verwendung nach Anspruch 5, worin der Destillationsrückstand mindestens eine der Verbindungen der Formeln 3-6 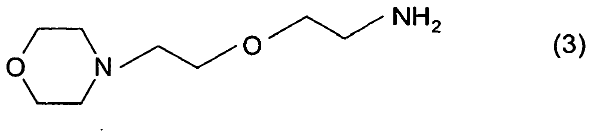 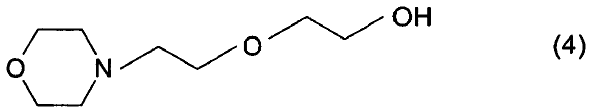 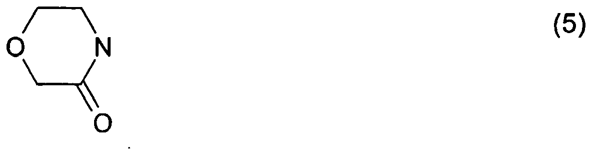 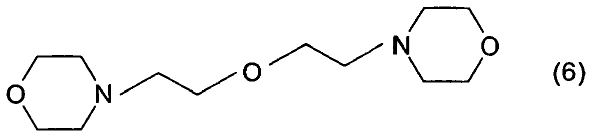 umfasst.

7. Verwendung nach einem oder mehreren der Ansprüche 1 bis 6 als Korrosionsinhibitoren an und in Einrichtungen zur Förderung und Transport von Kohlenwasserstoffen in der Erdölförderung und -verarbeitung.

8. Verwendung nach einem oder mehreren der Ansprüche 1 bis 6 als Korrosionsinhibitoren in Metallbearbeitungshilfsmitteln.

9. Salze aus Verbindungen der Formel (1) 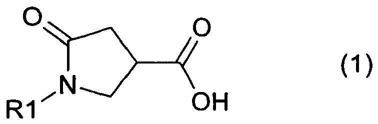 mit Aminen der Formel (2) 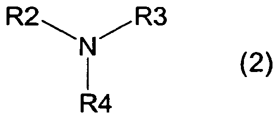 worin
R1 C₈- bis C₃₀-Alkyl oder C₈- bis C₃₀-Alkenyl
R2 Wasserstoff, C₁- bis C₆-Alkyl oder einen Heteroatome enthaltenden organischen Rest mit 1 bis 8 Kohlenstoffatomen, und,
R3, R4 unter Einschluss des Stickstoffatoms einen Cyclus mit 5 bis 7 Ringatomen bilden, wobei dieser Cyclus neben Kohlenstoffatomen gegebenenfalls ein Sauerstoffatom oder ein Stickstoffatom oder beides enthalten kann, bedeuten.

10. Salze nach Anspruch 9, worin die Amine der Formel 2 mindestens eine der Verbindungen der Formeln 3-6 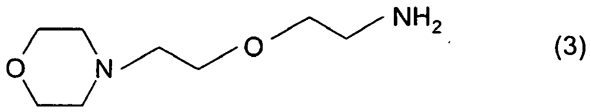 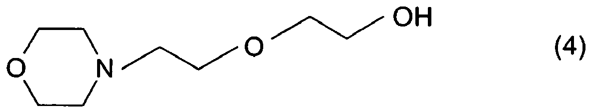 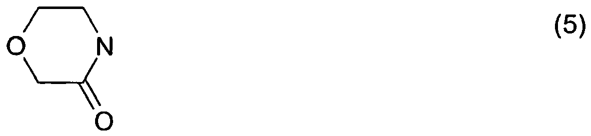 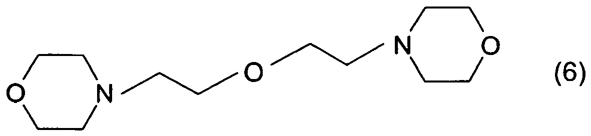 umfassen.

## Claims

1. The use of salts of compounds of the formula (1) 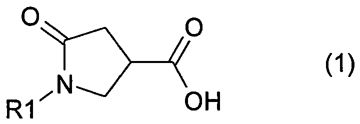 with amines of the formula (2) 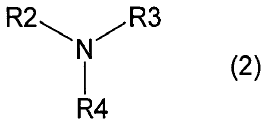 in which
R1 is C₈- to C₃₀-alkyl or C₈- to C₃₀-alkenyl
R2 is hydrogen, C₁- to C₆-alkyl or a heteroatom-containing organic radical having 1 to 8 carbon atoms, and
R3, R4 with inclusion of the nitrogen atom form a cycle having 5 to 7 ring atoms, where this cycle, in addition to carbon atoms, may optionally contain an oxygen atom or a nitrogen atom or both,
as water-soluble or water-dispersible corrosion inhibitors.

2. The use as claimed in claim 1, wherein R1 is an alkyl or alkenyl group of 8 to 18 carbon atoms.

3. The use as claimed in claim 1 and/or 2, wherein R2 is -CH₂-CH₂-OH.

4. The use as claimed in one or more of claims 1 to 3, in which the amine of the formula (2) is a compound of the formula (7) 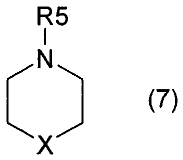 in which
R5 is hydrogen or an optionally heteroatom-containing C₁ to C₆ alkyl radical, and
X is C, O or N.

5. The use as claimed in one or more of claims 1 to 3, in which the amine of the formula 2 is a distillation residue from morpholine production.

6. The use as claimed in claim 5, in which the distillation residue comprises at least one of the compounds of the formulae 3-6

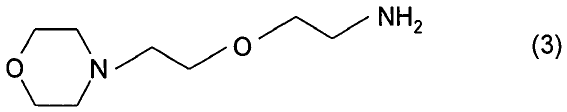

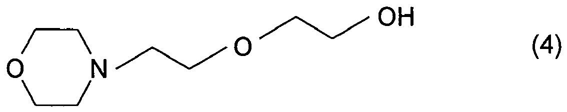

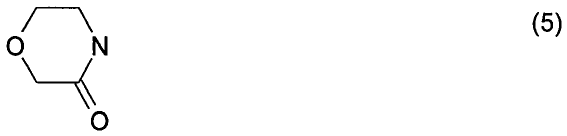

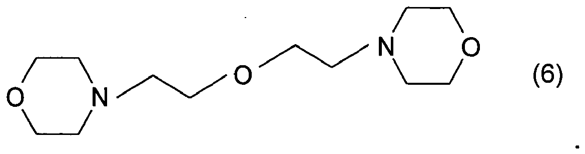

7. The use as claimed in one or more of claims 1 to 6 as corrosion inhibitors on and in devices for extraction and transport of hydrocarbons in mineral oil extraction and processing.

8. The use as claimed in one or more of claims 1 to 6 as corrosion inhibitors in metalworking assistants.

9. A salt of compounds of the formula (1) 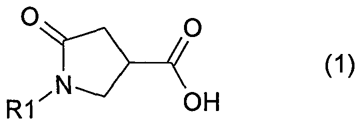 with amines of the formula (2) 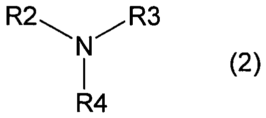 in which
R1 is C₈- to C₃₀-alkyl or C₈- to C₃₀-alkenyl
R2 is hydrogen, C₁- to C₆-alkyl or a heteroatom-containing organic radical having 1 to 8 carbon atoms, and
R3, R4 with inclusion of the nitrogen atom form a cycle having 5 to 7 ring atoms, where this cycle, in addition to carbon atoms, may optionally contain an oxygen atom or a nitrogen atom or both.

10. The salt as claimed in claim 9, wherein the amines of the formula 2 comprise at least one of the compounds of the formulae 3-6

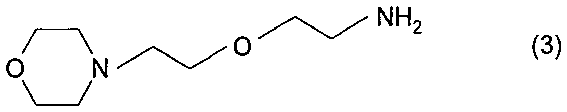

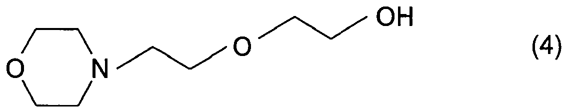

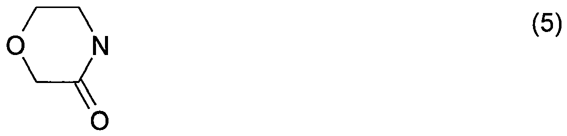

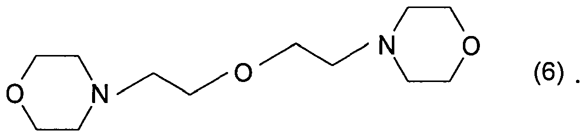

## Revendications

1. Utilisation de sels de composés de formule (1) 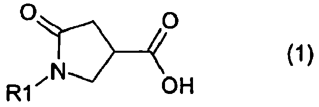 avec des amines de formule (2) 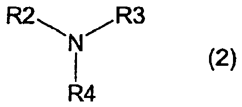 où
R1 signifie C₈-C₃₀-alkyle ou C₈-C₃₀-alcényle
R2 signifie hydrogène, C₁-C₆-alkyle ou un radical organique contenant des hétéroatomes comprenant 1 à 8 atomes de carbone, et
R3, R4 forment, en incluant l'atome d'azote, un cycle de 5 à 7 atomes de cycle, ce cycle pouvant contenir, outre des atomes de carbone, le cas échéant un atome d'oxygène ou un atome d'azote ou les deux,
comme inhibiteurs de corrosion solubles ou, selon le cas, dispersibles dans l'eau.

2. Utilisation selon la revendication 1, où R1 représente un groupe alkyle ou alcényle comprenant 8 à 18 atomes de carbone.

3. Utilisation selon la revendication 1 et/ou 2, où R2 représente -CH₂-CH₂-OH.

4. Utilisation selon l'une ou plusieurs des revendications 1 à 3, où l'amine de formule (2) représente un composé de formule (7) 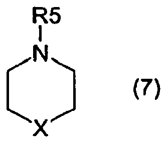 où
R5 signifie hydrogène ou un radical C₁-C₆-alkyle contenant le cas échéant des hétéroatomes et
X signifie C, O ou N.

5. Utilisation selon l'une ou plusieurs des revendications 1 à 3, où l'amine de formule 2 est un résidu de distillation de la préparation de morpholine.

6. Utilisation selon la revendication 5, où le résidu de distillation comprend au moins un des composés des formules 3-6

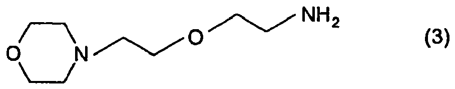

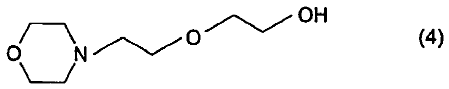

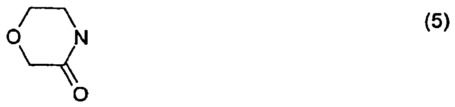

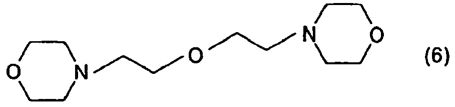

7. Utilisation selon l'une ou plusieurs des revendications 1 à 6 comme inhibiteurs de corrosion sur et dans des dispositifs d'extraction et de transport d'hydrocarbures dans l'extraction et la transformation de pétrole.

8. Utilisation selon l'une ou plusieurs des revendications 1 à 6 comme inhibiteurs de corrosion dans les adjuvants de traitement de métaux.

9. Sels de composés de formule (1) 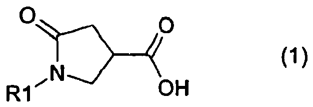 avec des amines de formule (2) 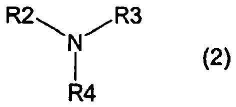 où
R1 signifie C₈-C₃₀-alkyle ou C₈-C₃₀-alcényle
R2 signifie hydrogène, C₁-C₆-alkyle ou un radical organique contenant des hétéroatomes comprenant 1 à 8 atomes de carbone, et
R3, R4 forment, en incluant l'atome d'azote, un cycle de 5 à 7 atomes de cycle, ce cycle pouvant contenir, outre des atomes de carbone, le cas échéant un atome d'oxygène ou un atome d'azote ou les deux.

10. Sels selon la revendication 9, où les amines de formule 2 englobent au moins un des composés des formules 3-6
